# EUROPEAN PATENT SPECIFICATION

(11) **EP 1 377 555 B1**
(45) Date of publication and mention of the grant of the patent: **24.01.2007**
(21) Application number: 02730147.2
(22) Date of filing: 11.04.2002
(51) Int. Cl.: C07D 215/52, C07D 401/14, A61K 31/47, C07D 405/14

(54) **A DIOXINO[2,3-G]QUINOLINE-9-CARBOXYLIC ACID DERIVATIVE AS NK3 RECEPTOR ANTAGONIST**
EIN DIOXINO[2,3-G]CHINOLIN-9-CARBONSÄURE DERIVAT ALS NK3 REZEPTOR ANTAGONIST
UN DERIVE DE DIOXINO[2,3-G]QUINOLINE-9-ACIDE CARBOXYLIQUE EN TANT QU'ANTAGONISTE DU RECEPTEUR NK3

(30) Priority: 11.04.2001 GB 0109122
(43) Date of publication of application: 07.01.2004
(73) Proprietor: GlaxoSmithKline S.p.A., 37135 Verona (IT)
(72) Inventor: FARINA, Carlo, c/o Nikem Research S.r.l., 20021 Baranzate di Bollate (IT); GIARDINA, Giuseppe Arnaldo Maria, Nikem Res S.r.l., 20021 Baranzate di Bollate (IT); GRUGNI, Mario, c/o Nikem Research S.r.l., 20021 Baranzate di Bollate (IT); PERUGINI, Lorenzo, c/o GlaxoSmithKline S.p.A., 37135 Verona (IT)
(74) Representative: Rutter, Keith
(86) International application number: PCT/EP2002/004069
(87) International publication number: WO 2002/083645

(56) References cited:
- WO-A-00/31037
- WO-A-00/31038
- WO-A-96/02509
- WO-A-97/19926
- WO-A-98/52942
- BLANEY ET AL: "Stepwise Modulation of Neurokinin-3 and Neurokinin-2 Receptor Affinity and Selectivity in Quinoline Tachykinin Receptor Antagonists" EUROPEAN JOURNAL OF MEDICINAL CHEMISTRY, EDITIONS SCIENTIFIQUE ELSEVIER, PARIS, FR, vol. 44, 2001, pages 1675-1689, XP002192370 ISSN: 0223-5234
- GIARDINA G A M ET AL: "DISCOVERY OF A NOVEL CLASS OF SELECTIVE NON-PEPTIDE ANTAGONISTS FOR THE HUMAN NEUROKININ-3 RECEPTOR. 2. IDENTIFICATION OF (S)-N- (1-PHENYLPROPYL)-3-HYDROXY-2-PHENYLQUINOLI NE-4-CARBOXAMIDE (SB 223412)" JOURNAL OF MEDICINAL CHEMISTRY, AMERICAN CHEMICAL SOCIETY. WASHINGTON, US, vol. 42, no. 6, 1999, pages 1053-1065, XP000882756 ISSN: 0022-2623
- GIARDINA G A M ET AL: "2-PHENYL-4-QUINOLINECARBOXYAMIDES: A NOVEL CLASS OF POTENT AND SELECTIVE NON-PEPTIDE COMPETITIVE ANTAGONISTS FOR THE HUMAN NEUROKININ-3 RECEPTOR" JOURNAL OF MEDICINAL CHEMISTRY, AMERICAN CHEMICAL SOCIETY. WASHINGTON, US, vol. 39, no. 12, 7 June 1996 (1996-06-07), pages 2281-2284, XP000197077 ISSN: 0022-2623

## Description

The present invention relates to the novel quinoline derivative 8-[1,4']Bipiperidinyl-1'-ylmethyl-7-phenyl-2,3-dihydro-[1,4]dioxino[2,3-g]quinoline-9-carboxylic acid ((S)-1-cyclohexyl-ethyl)-amide or a pharmaceutically acceptable salt or solvate thereof, to processes for the preparation of such a compound, to pharmaceutical compositions containing such a compound and to the use of such a compound in medicine.

The mammalian peptide Neurokinin B (NKB) belongs to the Tachykinin (TK) peptide family which also include Substance P (SP) and Neurokinin A (NKA). Pharmacological and molecular biological evidence has shown the existence of three subtypes of TK receptor (NK₁, NK₂ and NK₃) and NKB binds preferentially to the NK₃ receptor although it also recognises the other two receptors with lower affinity (Maggi et al, **1993,** *J. Auton. Pharmacol., 13,* 23-93).

Selective peptidic NK₃ receptor antagonists are known (Drapeau, **1990** *Regul. Pept.,* 31, 125-135), and findings with peptidic NK₃ receptor agonists suggest that NKB, by activating the NK₃ receptor, has a key role in the modulation of neural input in airways, skin, spinal cord and nigro-striatal pathways (Myers and Undem, 1993, *J.Physiol.,* 470, 665-679; Counture et al., 1993, *Regul. Peptides,* 46, 426-429; Mccarson and Krause, 1994, *J. Neurosci.,* 14 (2), 712-720; Arenas et al. 1991, *J.Neurosci.,* 11, 2332-8). However, the peptide-like nature of the known antagonists makes them likely to be too labile from a metabolic point of view to serve as practical therapeutic agents.

International Patent Application, Publication Number WO 00/58307 describes a series of aryl fused 2,4-disubstituted pyridines, such as naphthyridine derivatives, which are stated to exhibit biological activity as NK₃ receptor antagonists.

The compounds of the present invention are quinoline derivatives. Other quinoline derivatives have been described previously as selective NK₃ antagonists. For example, International Patent Application, Publication Numbers, WO 95/32948 and WO 96/02509 describe a series of selective and potent NK₃ receptor antagonists.

International Patent Application, Publication Number WO 00/64877 describes a series of 2-aminoquinolinecarboxamides as neurokinin receptor ligands.

International Patent Application, Publication Number, WO 00/58303 describes a series of 4-substituted quinoline derivatives which are stated to be NK₃ and/or GABA(A) receptor ligands. Such compounds are characterised by the presence of a nitrogen-containing heterocyclic moiety at the C(4) position of the quinoline ring.

International Patent Application, Publication Numbers, WO 97/21680, WO 98/52942, WO 00/31037 and WO 00/31038 describe compounds which have biological activity as combined NK₃ and NK₂ receptor antagonists.

Copending International Patent Application Numbers, PCT/EP01/13833, PCT/EP01/14140 and PCT/EP01/13832 also describe compounds that have biological activity as combined NK₃ and NK₂ receptor antagonists.

We have now discovered a further novel class of non-peptide NK₃ antagonists which are far more stable from a metabolic point of view than the known peptidic NK₃ receptor antagonists and are of potential therapeutic utility. These compounds also have NK₂ antagonist activity and are therefore considered to be of potential use in the prevention and treatment of a wide variety of clinical conditions, which are characterised by overstimulation of the Tachykinin receptors, in particular NK₃ and NK₂.

These conditions include respiratory diseases, such as chronic obstructive pulmonary disease (COPD), asthma, airway hyper-reactivity, cough; inflammatory diseases such as inflammatory bowel disease, psoriasis, fibrositis, osteoarthritis, rheumatoid arthritis and inflammatory pain; neurogenic inflammation or peripheral neuropathy, allergies such as eczema and rhinitis; ophthalmic diseases such as ocular inflammation, conjunctivitis, vernal conjuctivitis and the like; cutaneous diseases, skin disorders and itch, such as cutaneous wheal and flare, contact dermatitis, atopic dermatitis, urticaria and other eczematoid dermatitis; adverse immunological reactions such as rejection of transplanted tissues and disorders related to immune enhancement or suppression such as systhemic lupus erythematosis; gastrointestinal (GI) disorders and diseases of the GI tract such as disorders associated with the neuronal control of viscera such as ulcerative colitis, Crohn's disease, irritable bowel syndrome (IBS), gastro-exophageous reflex disease (GERD); urinary incontinence and disorders of the bladder function; renal disorders; increased blood pressure, proteinuria, coagulopathy and peripheral and cerebral oedema following pre-eclaympsia in pregnancies (hereinafter referred to as the 'Primary Conditions').

Certain of these compounds also show CNS activity and hence are considered to be of particular use in the treatment of disorders of the central nervous system such as anxiety, depression, psychosis and schizophrenia; neurodegenerative disorders such as AIDS related dementia, senile dementia of the Alzheimer type, Alzheimer's disease, Down's syndrome, Huntingdon's disease, Parkinson's disease, movement disorders and convulsive disorders (for example epilepsy); demyelinating diseases such as multiple sclerosis and amyotrophic lateral sclerosis and other neuropathological disorders such as diabetic neuropathy, AIDS related neuropathy, chemotherapy-induced neuropathy and neuralgia; addiction disorders such as alcoholism; stress related somatic disorders; reflex sympathetic dystrophy such as shoulder/hand syndrome; dysthymic disorders; eating disorders (such as food intake disease); fibrosing and collagen diseases such as scleroderma and eosinophilic fascioliasis; disorders of the blood flow caused by vasodilatation and vasospastic diseases such as angina, migraine and Reynaud's disease and pain or nociception, for example, that is attributable to or associated with any of the foregoing conditions especially the transmission of pain in migraine, (hereinafter referred to as the 'Secondary Conditions').

The compounds of formula (I) are also considered to be useful as diagnostic tools for assessing the degree to which neurokinin-3 and neurokinin-2 receptor activity (normal, overactivity or underactivity) is implicated in a patient's symptoms.

Certain compounds of the present invention have also been found to exhibit surprisingly advantageous pharmacochemical properties.

According to the present invention, there is provided a compound of formula (I) below: 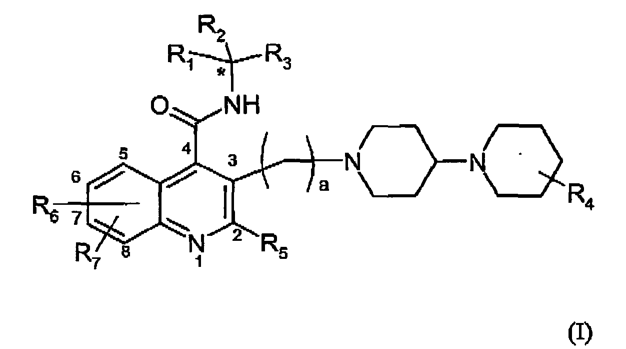 said compound being 8-[1,4']Bipiperidinyl-1'-ylmethyl-7-phenyl-2,3-dihydro-[1,4]dioxino[2,3-g]quinoline-9-carboxylic acid ((S)-1-cyclohexyl-ethyl)-amide or a pharmaceutically acceptable salt or solvate thereof.

The compounds of formula (I) may have at least one asymmetric centre - for example the carbon atom labelled with an asterisk (*) in the compound of formula (I) - and therefore may exist in more than one stereoisomeric form. The invention extends to all such stereoisomeric forms and to mixtures thereof, including racemates. In particular, the invention includes compounds wherein the asterisked carbon atom in formula (I) has the stereochemistry shown in formula (Ia): 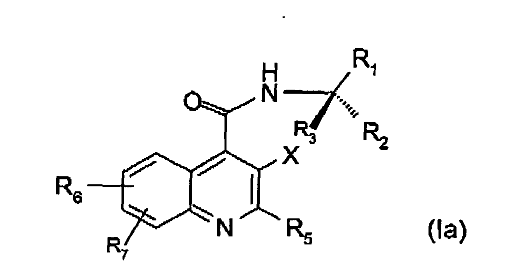 wherein R₁, R₂, R₃, R₅, R₆, and R₇ are as defined in relation to formula (I), and X represents the moiety

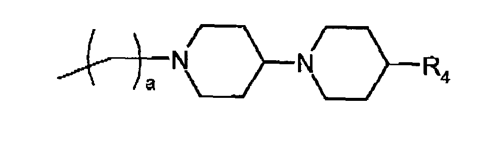

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels.

A substantially pure form will generally contain at least 50% (excluding normal pharmaceutical additives), preferably 75%, more preferably 90% and still more preferably 95% of the compound of formula (I) or its salt or solvate.

One preferred pharmaceutically acceptable form is the crystalline form, including such form in pharmaceutical composition. In the case of salts and solvates the additional ionic and solvent moieties must also be non-toxic.

Suitable salts are pharmaceutically acceptable salts.

Suitable pharmaceutically acceptable salts include the acid addition salts with the conventional pharmaceutical acids, for example maleic, hydrochloric, hydrobromic, phosphoric, acetic, fumaric, salicylic, citric, lactic, mandelic, tartaric, succinic, benzoic, ascorbic and methanesulphonic.

Suitable pharmaceutically acceptable salts include salts of acidic moieties of the compounds of formula (I) when they are present, for example salts of carboxy groups or phenolic hydroxy groups.

Suitable salts of acidic moieties include metal salts, such as for example aluminium, alkali metal salts such as lithium, sodium or potassium, alkaline earth metal salts such as calcium or magnesium and ammonium or substituted ammonium salts, for example those with lower alkylamines such as triethylamine, hydroxy alkylamines such as 2-hydroxyethylamine, bis-(2-hydroxyethyl)-amine or tri-(2-hydroxyethyl)-amine, cycloalkylamines such as bicyclohexylamine, or with procaine, dibenzylpiperidine, N-benzyl-β-phenethylamine, dehydroabietylamine, N,N'-bisdehydroabietylamine, glucamine, N-methylglucamine or bases of the pyridine type such as pyridine, collidine, quinine or quinoline.

Suitable solvates are pharmaceutically acceptable solvates.

Suitable pharmaceutically acceptable solvates include hydrates.

The invention also provides a process for the preparation of a compound of formula (I), or a salt thereof and/or a solvate thereof, which process comprises reacting a compound of formula (II) or an active derivative thereof: 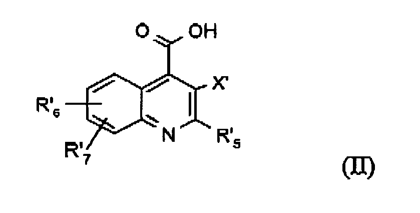 wherein R'₆, R'₇, R'₅ and X' are R₆, R₇, R₅ and X respectively as hereinbefore defined in relation to formula (I) or (Ia), or a group convertible to R₆, R₇, R₅ and X respectively; with a compound of formula (III): 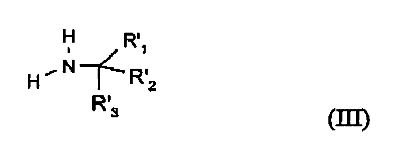 wherein R₁', R₂', and R₃' are R₁, R₂, and R₃ as defined for formula (I) or a group or atom convertible to R₁, R₂, and R₃ respectively; to form a compound of formula (Ib): 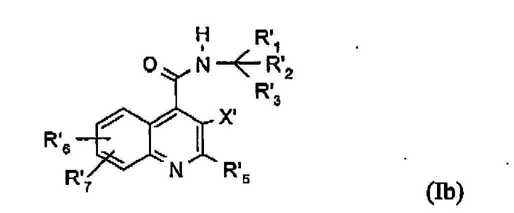 wherein R'₁, R'₂, R'₃, X', R'₅, R'₆ and R'₇ are as defined above, and thereafter carrying out one or more of the following optional steps:
(i) converting any one of R'₁, R'₂, R'₃, X', R'₅, R'₆ and R'₇ to R₁, R₂, R₃, X, R₅, R₆ and R₇ respectively as required, to obtain a compound of formula (I);
(ii) converting a compound of formula (I) into another compound of formula (I); and
(iii) preparing a salt of the compound of formula (I) and/or a solvate thereof.

Suitable groups convertible into other groups include protected forms of said groups.

Suitably R'₁, R'₂, R'₃, X', R'₅, R'₆ and R'₇ each represents R₁, R₂, R₃, X, R₅, R₆ and R₇ respectively or a protected form thereof.

It is favoured if the compound of formula (II) is present as an active derivative.

A suitable active derivative of a compound of formula (II) is a transient activated form of the compound of formula (II) or a derivative wherein the carboxy group of the compound of formula (II) has been replaced by a different group or atom, for example by an acyl halide, preferably a chloride, or an acylazide or a carboxylic acid anhydride.

Other suitable active derivatives include: a mixed anhydride formed between the carboxyl moiety of the compound of formula (II) and an alkyl chloroformate; an activated ester, such as a cyanomethyl ester, thiophenyl ester, p-nitrophenyl ester, p-nitrothiophenyl ester, 2,4,6-trichlorophenyl ester, pentachlorophenyl ester, pentafluorophenyl ester, N-hydroxy-phtalimido ester, N-hydroxypiperidine ester, N-hydroxysuccinimide ester, N-hydroxy benzotriazole ester; alternatively, the carboxy group of the compound of formula (II) may be activated using a carbodiimide or N,N'-carbonyldiimidazole.

The reaction between the compound of formula (II) or the active derivative thereof and the compound of formula (III) is carried out under the appropriate conventional conditions for the particular compounds chosen. Generally, when the compound of formula (II) is present as an active derivative the reaction is carried out using the same solvent and conditions as used to prepare the active derivative, preferably the active derivative is prepared *in situ* prior to forming the compound of formula (Ib) and thereafter the compound of formula (I) or a salt thereof and/or a solvate thereof is prepared.

For example, the reaction between an active derivative of the compound of formula (II) and the compound of formula (III) may be carried out:
(a) by first preparing an acid chloride and then coupling said chloride with the compound of formula (III) in the presence of an inorganic or organic base in a suitable aprotic solvent such as dimethylformamide (DMF) at a temperature in a range from -70 to 50°C (preferably in a range from -10 to 20°C); or
(b) by treating the compound of formula (II) with a compound of formula (III) in the presence of a suitable condensing agent, such as for example N,N'-carbonyl diimidazole (GDI) or a carbodiimide such as dicyclohexylcarbodiimide (DCC) or N-dimethylaminopropyl-N'-ethylcarbodiimide, preferably in the presence of N-hydroxybenzotriazole (HOBT) to maximise yields and avoid racemization processes (see *Synthesis,* 453, 1972), or O-benzotriazol-1-yl-N,N,N',N'-tetramethyluroniumhexafluorophosphate (HBTU), in an aprotic solvent, such as a mixture of acetonitrile (MeCN) and tetrahydrofuran (THF), for example a mixture in a volume ratio of from 1:9 to 7:3 (MeCN:THF), at any temperature providing a suitable rate of formation of the required product, such as a temperature in the range of from -70 to 50°C, preferably in a range of from -10 to 25°C, for example at 0°C.

A preferred reaction is set out in Scheme la shown below: 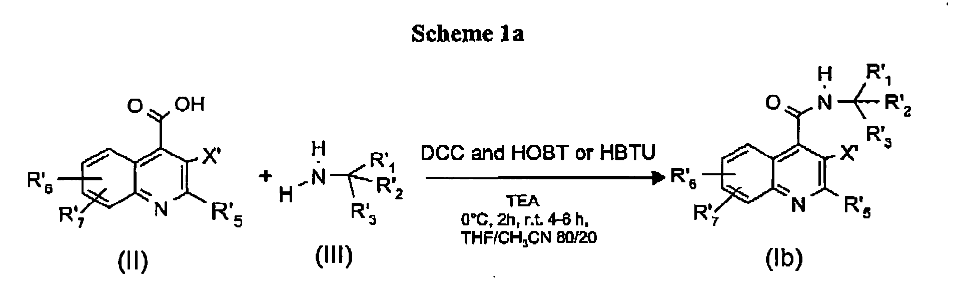 wherein R'₁, R'₂, R'₃, X', R'₅, R'₆ and R'₇ are as defined above.

Another preferred reaction is set out in Scheme 1b shown below: 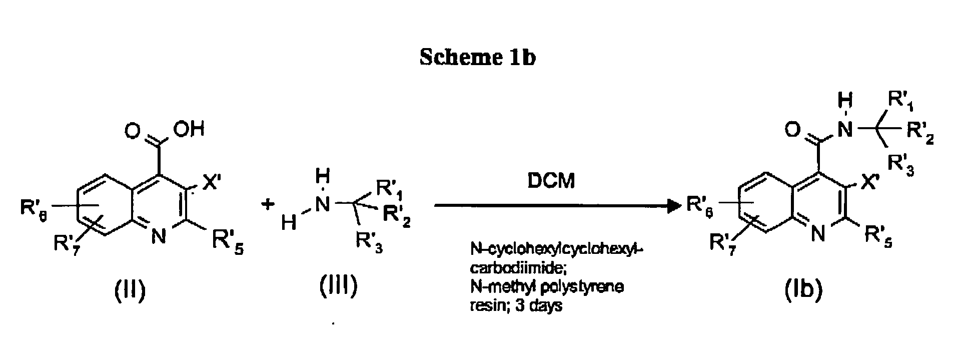 wherein R'₁, R'₂, R'₃, X', R'₅, R'₆ and R'₇ are as defined above.

It will be appreciated that a compound of formula (Ib) may be converted to a compound of formula (I), or one compound of formula (I) may be converted to another compound of formula (I) by interconversion of suitable substituents. Thus, certain compounds of formula (I) and (Ib) are useful intermediates in forming other compounds of the present invention.

Accordingly, in a further aspect the invention provides a process for preparing a compound of formula (I), or a salt thereof and/or a solvate thereof, which process comprises converting a compound of the above defined formula (Ib) wherein at least one of R'₁, R'₂, R'₃, X', R'₅, R'₆ and R'₇ is not R₁, R₂, R₃, X, R₅, R₆ or R₇ respectively, thereby to provide a compound of formula (I); and thereafter, as required, carrying out one or more of the following optional steps:
(i) converting a compound of formula (I) into another compound of formula (I); and
(ii) preparing a salt of the compound of formula (I) and/or a solvate thereof.

Suitably, in the compound of formula (Ib) the variables R'₁, R'₂, R'₃, X', R'₅, R'₆ and R'₇ are R₁, R₂, R₃, X, R₅, R₆ and R₇ respectively or they are protected forms thereof

The above mentioned conversions, protections and deprotections are carried out using the appropriate conventional reagents and conditions and are further discussed below.

In some embodiments, a compound of formula (II) or the corresponding alkyl (such as methyl or ethyl) ester, may be prepared by reacting a compound of formula (IV) or the corresponding alkyl (such as methyl or ethyl) ester: 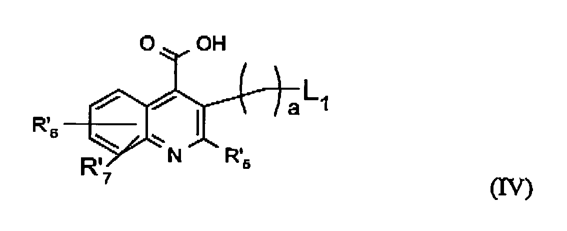 wherein R'₆, R'₇, R'₅ and a are as defined above and L₁ represents a halogen atom such as a bromine atom, with a compound of formula (V): 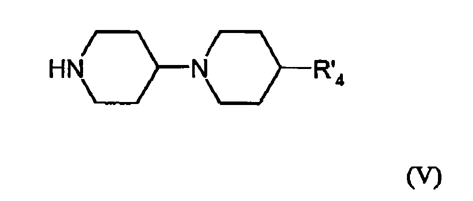 wherein R'₄ is R₄ as defined in relation to formula (I) or a protected form thereof.

Suitably, R'₄ is R₄.

Suitably, reaction between the compounds of formulae (IV) or the corresponding alkyl (such as methyl or ethyl) ester and (V) is carried out under conventional amination conditions, for example when L₁ is a bromine atom then the reaction is conveniently carried out in an aprotic solvent, such as tetrahydrofuran or dimethylformamide at any temperature providing a suitable rate of formation of the required product, usually at ambient temperature; preferably the reaction is carried out in the presence of triethylamine (TEA) or K₂CO₃.

Where a is 1, a compound of formula (IV) or the corresponding alkyl (such as methyl or ethyl) ester can be prepared by appropriate halogenation of a compound of formula (VI) or the corresponding alkyl (such as methyl or ethyl) ester: 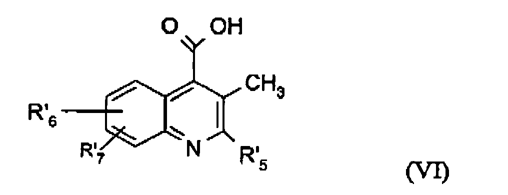 wherein R'₆, R'₇ and R'₅ are as defined above in relation to formula (II).

Suitable halogenation reagents are conventional reagents depending upon the nature of the halogen atom required, for example when L₁ is bromine a preferred halogenation reagent is N-bromosuccinimide (NBS).

The halogenation of the compound of formula (VI) or the corresponding alkyl (such as methyl or ethyl) ester is preferably carried out under conventional conditions, for example bromination is carried out by treatment with NBS in an inert solvent, such as carbon tetrachloride CCl₄, or 1,2-dichloroethane or CH₃CN, at any temperature providing a suitable rate of formation of the required product, suitably at an elevated temperature such as a temperature in the range of 60°C to 100°C, for example 80°C; preferably the reaction is carried out in the presence of a catalytic amount of benzoyl peroxide.

A compound of formula (VI) may conveniently be prepared by reacting a compound of formula (A) 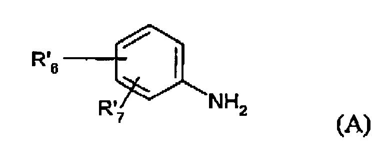 wherein R'₆ and R'₇ are as defined in relation to formula (II), with a compound of formula (B):

R'₅-CHO (B)

wherein R'₅ is as defined in relation to formula (II), in the presence of oxobutyric acid.

The reaction between the compounds of formula (A) and (B) respectively is conveniently carried out using Doebner-Miller reaction conditions (see for example Chem. Ber. 29, 352 (1894); Chem. Revs. 35, 153, (1944); J. Chem. Soc. B, 1969, 805), for example in an alcoholic solvent such as ethanol, at any temperature providing a suitable rate of formation of the required product, but generally at an elevated temperature, such as the reflux temperature of the solvent.

The compounds of formula (A) and (B) are known compounds or they are prepared according to methods used to prepare known compounds for example as described in *Vogel's Textbook of Practical Organic Chemistry.*

Alternatively a compound of formula (VI) can be prepared by reacting a compound of formula (VII) 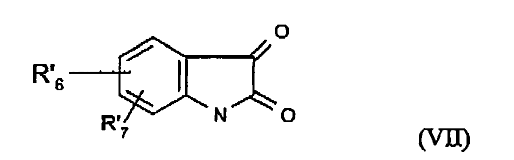 wherein R'₆ and R'₇ are as defined in relation to formula (II), with a compound of formula (C):

R₅'-CO-CH₂-CH₃ (C)

The reaction between the compounds of formula (VII) and (C) respectively is conveniently carried out using Pfitzinger reaction conditions (see for example J. Prakt. Chem. 33, 100 (1886), J. Prakt. Chem. 38, 582 (1888), J. Chem. Soc. 106 (1948) and Chem. Rev. 35, 152 (1944)), for example in an alkanolic solvent such as ethanol, at any temperature providing a suitable rate of formation of the required product, but generally at an elevated temperature, such as the reflux temperature of the solvent, and preferably in the presence of a base such as potassium hydroxide or potassium tert-butoxide. The compounds of formula (C) are known compounds or they are prepared according to methods used to prepare known compounds for example as described in *Vogel's Textbook of Practical Organic Chemistry.*

In the case in which the corresponding alkyl (such as methyl or ethyl) ester of compounds (VI), (IV) and (II) are utilised, a hydrolysis to compound (II) is required before conversion to compound (Ib) in Scheme 1a or 1b. Such hydrolysis can be carried out under acidic conditions, such 10-36% hydrochloric acid at a temperature in the range between 30 and 100 °C.

In other embodiments, a compound of formula (IT) wherein X' represents 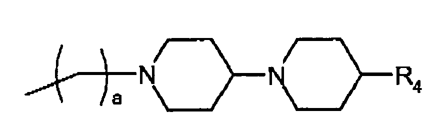 may conveniently be prepared by reacting a compound of formula (VII) with a compound of formula (VIII):

R₅'-CO-CH₂-(CH₂)a-T₅ (VIII)

wherein R'₅ and a are as defined in relation to formula (II), and T₅ is a group X' as defined in relation to formula (II) or a protected form thereof or a group convertible thereto; and thereafter as required removing any protecting group, for example by dehydrogenation, and/or converting any group T₅ to X as defined in relation to formula (I).

The reaction between the compounds of formula (VII) and (VIII) is conveniently carried out using Pfitzinger reaction conditions (see for example J. Prakt. Chem. 33, 100 (1886), J. Prakt. Chem. 38, 582 (1888), J. Chem. Soc. 106 (1948) and Chem. Rev. 35, 152 (1944)), for example in an alkanolic solvent such as ethanol, at any temperature providing a suitable rate of formation of the required product, but generally at an elevated temperature, such as the reflux temperature of the solvent, and preferably in the presence of a base such as potassium hydroxide or potassium tert-butoxide.

Protected forms of 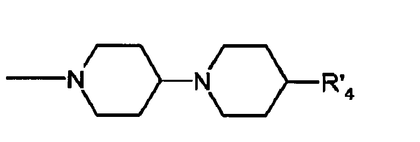 will vary according to the particular nature of the group being protected but will be chosen in accordance with normal chemical practice.

Groups convertible to 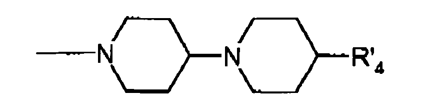 include groups dictated by conventional chemical practice to be required and to be appropriate, depending upon the specific nature of the 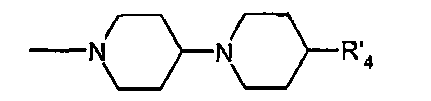 under consideration.

Suitable deprotection methods for deprotecting protected forms of 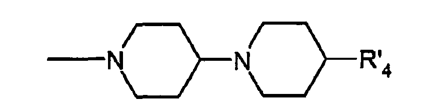 and conversion methods for converting T₅ to 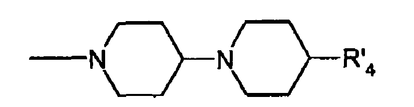 will be those used conventionally in the art depending upon the particular groups under consideration with reference to standard texts such as Greene, T.W. and Wuts, P.G.M. Protective Groups in Organic Synthesis, John Wiley & Sons Inc. New York, 1991 (Second Edt.) or in Kocienski, P.J. Protecting groups. George Thieme Verlag, New York, 1994 and Chemistry of the Amino Group, Patais (Ed.), Interscience, New York 1968; or Advanced Organic Chemistry, March J, John Wiley & Sons, New York, 1992.

A compound of formula (VIII) is prepared from a compound of formula (IX):

R₅'-CO-CH₂-(CH₂)ₐ-OH (IX)

wherein R'₅ is as defined in relation to formula (II) and a is as defined in relation to formula (VIII), by first halogenating, preferably brominating, or mesylating the compound of formula (IX) and thereafter reacting the halogenation or mesylation product so formed with a compound capable of forming a group T₅ so as to provide the required compound of formula (VII).

When T₅ is a group 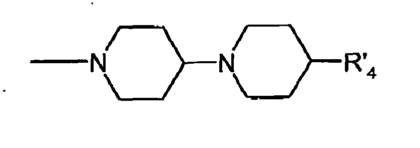 a compound capable of forming a group T₅ is a compound of the above defined formula (V).

The halogenation of the compound of formula (IX) is suitably carried out using a conventional halogenation reagent. Mesylation is conveniently carried out using mesyl chloride in an inert solvent such as methylene dichloride, at a temperature below room temperature, such as 0°C, preferably in the presence of triethylamine. The reaction conditions between the compound of formula (IX) and the compound capable of forming a group T₅ will be those conventional conditions dictated by the specific nature of the reactants, for example when the T₅ required is a group 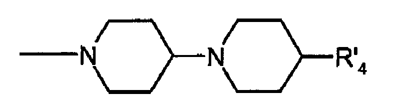 and the required compound capable of forming a group T₅ is a compound of the above defined formula (V), then the reaction between the halogenation or mesylation product of the compound of formula (IX) and the compound of formula (V) is carried out under analogous conditions to those described for the reaction between the compounds of formulae (IV) and (V).

Other compounds capable of forming a group T₅ will depend upon the particular nature of T₅, but will be those appropriate compounds dictated by conventional chemical practice with reference to standard texts such as Chemistry of the Amino Group, Patais (Ed.), Interscience, New York 1968; and Advanced Organic Chemistry, March J, John Wiley & Sons, New York, 1992.

A compound of formula (IX) may be prepared by reacting a compound of formula (X): 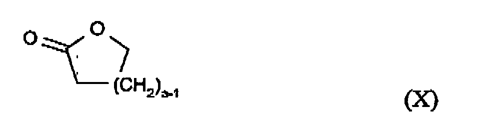 wherein a is as defined in relation to formula (VIII), with a lithium salt of formula (XI):

R'₅ Li (XI)

wherein R'₅ is as defined in relation to formula (II).

The reaction between the compounds of formulae (X) and (XI) can be carried out in an aprotic solvent, such as diethyl-ether at any temperature providing a suitable rate of formation of the required product, usually at a low temperature such as in the range of - 10°C to -30°C, for example -20°C.

The compounds of formula (HI) are known commercially available compounds or they can be prepared from known compounds by known methods, or methods analogous to those used to prepare known compounds, for example the methods described in Liebigs Ann. der Chemie, (1936), 523, 199.

A chiral compound of formula (III) wherein R₂ is a C₅ or C₇ cycloalkyl group, R₃ is methyl and R₁ is H are described in J. Org. Chem. (1996), 61 (12), 4130-4135. A chiral compound of formula (III) wherein R₂ is phenyl, R₃ is isopropyl and R₁ is H is a known compound described in for example Tetrahedron Lett. (1994), 35(22), 3745-6.

The compounds of formula (V) are known, commercially available compounds or they can be prepared using methods analogous to those used to prepare known compounds; for example the methods described in the Chemistry of the Amino Group, Patais (Ed.), Interscience, New York 1968; Advanced Organic Chemistry, March J, John Wiley & Sons, New York, 1992 ; J. Heterocyclic Chem. (1990), 27, 1559; Synthesis (1975), 135, Bioorg. Med. Chem. Lett. (1997), 7, 555, or Protective Groups in Organic Synthesis (second edition), Wiley Interscience, (1991) or other methods mentioned herein.

The compounds of formula (VII) are known compounds or they are prepared according to methods used to prepare known compounds for example those disclosed in J. Org. Chem. 21, 171 (1955); J. Org. Chem. 21, 169 (1955).

The compounds of formula (X) and (XI) are known compounds or they are prepared according to methods used to prepare known compounds for example those disclosed by Krow G. R. in Organic Reactions, Vol 43, page 251, John Wiley & Sons Inc.1994 (for the compounds of formula (X)) and Organometallics in Synthesis, Schlosser M.(Ed), John Wiley & Sons Inc. 1994 (for the compounds of formula (XI)).

As hereinbefore mentioned, the compounds of formula (I) may exist in more than one stereoisomeric form - and the process of the invention may produce racemates as well as enantiomerically pure forms. Accordingly, a pure enantiomer of a compound of formula (I) is obtained by reacting a compound of the above defined formula (II) with an appropriate enantiomerically pure primary amine of formula (IIIa) or (IIIc): 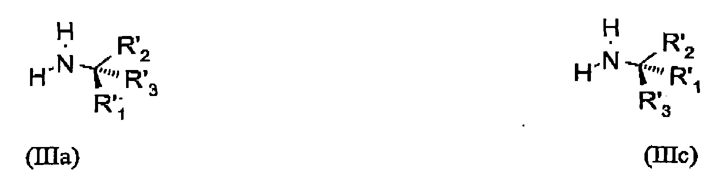 wherein R'₁, R'₂ and R'₃ are as defined above, to obtain a compound of formula (I'a) or (I'c): 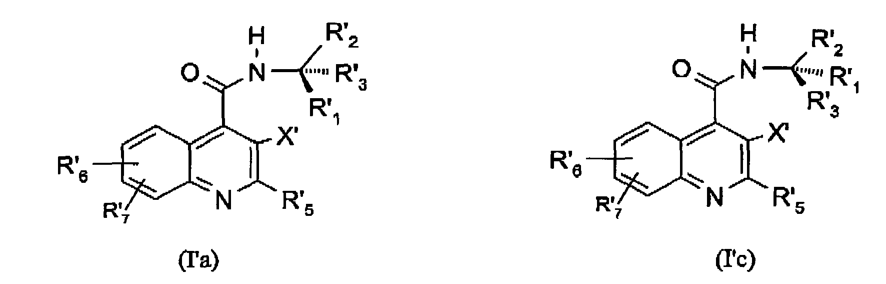 wherein R'₁, R'₂, R'₃, X', R'₅, R'₆, and R'₇ are as defined above.

Compounds of formula (I'a) or (I'c) may subsequently be converted to compounds of formula (Ia) or (Ic) by the methods of conversion mentioned before: 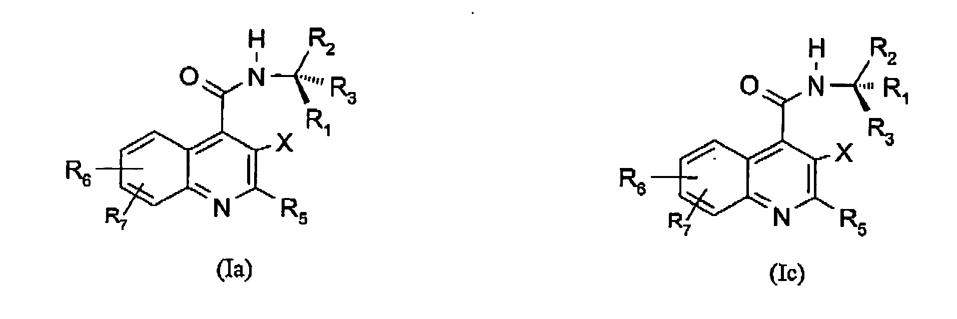 wherein R₁, R₂, R₃, X, R₅, R₆, and R₇ are as defined above.

Suitably, in the above mentioned compounds of formulae (Ia), (Ic), (Ia), (Ic), (IIIa) and (IIIc) R₁ represents hydrogen.

An alternative method for separating optical isomers is to use conventional, fractional separation methods in particular fractional crystallisation methods. Thus, a pure enantiomer of a compound of formula (I) is obtained by fractional crystallisation of a diastereomeric salt formed by reaction of the racemic compound of formula (I) with an optically active strong acid resolving agent, such as camphosulphonic acid, tartaric acid, O,O'-di-p-toluoyltartaric acid or mandelic acid, in an appropriate alcoholic solvent, such as ethanol or methanol, or in a ketonic solvent, such as acetone. The salt formation process should be conducted at a temperature between 20°C and 80°C, preferably at 50°C.

A suitable conversion of one compound of formula (I) into a further compound of formula (I) involves converting one group X into another group X by for example:
(i) converting a ketal into a ketone, by such as mild acidic hydrolysis, using for example dilute hydrochloric acid;
(ii) reducing a ketone to a hydroxyl group by use of a borohydride reducing agent;
(iii) converting a carboxylic ester group into a carboxyl group using basic hydrolysis; and/or
(iv) reducing a carboxylic ester group to a hydroxymethyl group, by use of a borohydride reducing agent.

As indicated above, where necessary, the conversion of any group R'₁, R'₂, R'₃, X', R'₅, R'₆, and R'₇ into R₁, R₂, R₃, X, R₅, R₆, and R₇ which as stated above are usually protected forms of R₁, R₂, R₃, X, R₅, R₆, or R₇ may be carried out using appropriate conventional conditions such as the appropriate deprotection procedure.

It will be appreciated that in any of the above mentioned reactions any reactive group in the substrate molecule may be protected and deprotected according to conventional chemical practice, for example as described by Greene, T.W. and Wuts, P.G.M. Protective Groups in Organic Synthesis, John Wiley & Sons Inc. New York, 1991 (Second Edt.) or in Kocienski, P.J. Protecting groups. George Thieme Verlag, New York, 1994.

Suitable protecting groups in any of the above mentioned reactions are those used conventionally in the art. Thus, for example suitable hydroxyl protecting groups include benzyl or trialkylsilyl groups.

The methods of formation and removal of such protecting groups are those conventional methods appropriate to the molecule being protected. Thus for example a benzyloxy group may be prepared by treatment of the appropriate compound with a benzyl halide, such as benzyl bromide, and thereafter, if required, the benzyl group may be conveniently removed using catalytic hydrogenation or a mild ether cleavage reagent such as trimethylsilyl iodide or boron tribromide.

As indicated above, the compounds of formula (I) have useful pharmaceutical properties.

Accordingly the present invention also provides a compound of formula (I), or a pharmaceutically acceptable salt or solvate thereof, for use as an active therapeutic substance.

In particular, the present invention also provides a compound of formula (I), or a pharmaceutically acceptable salt or solvate thereof, for the treatment or prophylaxis of the Primary and Secondary Conditions.

The present invention further provides a pharmaceutical composition comprising a compound of formula (I), or a pharmaceutically acceptable salt or solvate thereof, and a pharmaceutically acceptable carrier.

The present invention also provides the use of a compound of formula (I), or a pharmaceutically acceptable salt or solvate thereof, in the manufacture of a medicament for the treatment of the Primary and Secondary Conditions.

As mentioned above the Primary conditions include respiratory diseases, such as chronic obstructive pulmonary disease (COPD), asthma, airway hyperreactivity, cough; inflammatory diseases such as inflammatory bowel disease, psoriasis, fibrositis, osteoarthritis, rheumatoid arthritis and inflammatory pain; neurogenic inflammation or peripheral neuropathy, allergies such as eczema and rhinitis; ophthalmic diseases such as ocular inflammation, conjunctivitis, vernal conjuctivitis and the like; cutaneous diseases, skin disorders and itch, such as cutaneous wheal and flare, contact dermatitis, atopic dermatitis, urticaria and other eczematoid dermatitis; adverse immunological reactions such as rejection of transplanted tissues and disorders related to immune enhancement or suppression such as systhemic lupus erythematosis; gastrointestinal (GI) disorders and diseases of the GI tract such as disorders associated with the neuronal control of viscera such as ulcerative colitis, Crohn's disease, irritable bowel syndrome (IBS), gastro-exophageous reflex disease (GERD); urinary incontinence and disorders of the bladder function; renal disorders; increased blood pressure, proteinuria, coagulopathy and peripheral and cerebral oedema following pre-eclampsia in pregnancies.

As mentioned above, the Secondary conditions include disorders of the central nervous system such as anxiety, depression, psychosis and schizophrenia; neurodegenerative disorders such as AIDS related dementia, senile dementia of the Alzheimer type, Alzheimer's disease, Down's syndrome, Huntingdon's disease, Parkinson's disease, movement disorders and convulsive disorders (for example epilepsy); demyelinating diseases such as multiple sclerosis and amyotrophic lateral sclerosis and other neuropathological disorders such as diabetic neuropathy, AIDS related neuropathy, chemotherapy-induced neuropathy and neuralgia; addiction disorders such as alcoholism; stress related somatic disorders; reflex sympathetic dystrophy such as shoulder/hand syndrome; dysthymic disorders; eating disorders (such as food intake disease); fibrosing and collagen diseases such as scleroderma and eosinophilic fascioliasis; disorders of the blood flow caused by vasodilatation and vasospastic diseases such as angina, migraine and Reynaud's disease and pain or nociception, for example, that is attributable to or associated with any of the foregoing conditions especially the transmission of pain in migraine.

Such a medicament, and a composition of this invention, may be prepared by admixture of a compound of the invention with an appropriate carrier. It may contain a diluent, binder, filler, disintegrant, flavouring agent, colouring agent, lubricant or preservative in conventional manner.

These conventional excipients may be employed for example as in the preparation of compositions of known agents for treating the conditions.

Preferably, a pharmaceutical composition of the invention is in unit dosage form and in a form adapted for use in the medical or veterinarial fields. For example, such preparations may be in a pack form accompanied by written or printed instructions for use as an agent in the treatment of the conditions.

The suitable dosage range for the compounds of the invention depends on the compound to be employed and on the condition of the patient. It will also depend, inter alia, upon the relation of potency to absorbability and the frequency and route of administration.

The compound or composition of the invention may be formulated for administration by any route, and is preferably in unit dosage form or in a form that a human patient may administer to himself in a single dosage. Advantageously, the composition is suitable for oral, rectal, topical, parenteral, intravenous or intramuscular administration. Preparations may be designed to give slow release of the active ingredient.

Compositions may, for example, be in the form of tablets, capsules, sachets, vials, powders, granules, lozenges, reconstitutable powders, or liquid preparations, for example solutions or suspensions, or suppositories.

The compositions, for example those suitable for oral administration, may contain conventional excipients such as binding agents, for example syrup, acacia, gelatine, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinyl-pyrrolidone, sodium starch glycollate or microcrystalline cellulose; or pharmaceutically acceptable setting agents such as sodium lauryl sulphate.

Solid compositions may be obtained by conventional methods of blending, filling, tabletting or the like. Repeated blending operations may be used to distribute the active agent throughout those compositions employing large quantities of fillers. When the composition is in the form of a tablet, powder, or lozenge, any carrier suitable for formulating solid pharmaceutical compositions may be used, examples being magnesium stearate, starch, glucose, lactose, sucrose, rice flour and chalk. Tablets may be coated according to methods well known in normal pharmaceutical practice, in particular with an enteric coating. The composition may also be in the form of an ingestible capsule, for example of gelatine containing the compound, if desired with a carrier or other excipients.

Compositions for oral administration as liquids may be in the form of, for example, emulsions, syrups, or elixirs, or may be presented as a dry product for reconstitution with water or other suitable vehicle before use. Such liquid compositions may contain conventional additives such as suspending agents, for example sorbitol, syrup, methyl cellulose, gelatine, hydroxyethylcellulose, carboxymethylcellulose, aluminium stearate gel, hydrogenated edible fats; emulsifying agents, for example lecithin, sorbitan monooleate, or acacia; aqueous or non-aqueous vehicles, which include edible oils; for example almond oil, fractionated coconut oil, oily esters, for example esters of glycerine, or propylene glycol, or ethyl alcohol, glycerine, water or normal saline; preservatives, for example methyl or propyl p-hydroxybenzoate or sorbic acid; and if desired conventional flavouring or colouring agents.

The compounds of this invention may also be administered by a non-oral route. In accordance with routine pharmaceutical procedure, the compositions may be formulated, for example for rectal administration as a suppository. They may also be formulated for presentation in an injectable form in an aqueous or non-aqueous solution, suspension or emulsion in a pharmaceutically acceptable liquid, e.g. sterile pyrogen-free water or a parenterally acceptable oil or a mixture of liquids. The liquid may contain bacteriostatic agents, anti-oxidants or other preservatives, buffers or solutes to render the solution isotonic with the blood, thickening agents, suspending agents or other pharmaceutically acceptable additives. Such forms will be presented in unit dose form such as ampoules or disposable injection devices or in multi- dose forms such as a bottle from which the appropriate dose may be withdrawn or a solid form or concentrate which can be used to prepare an injectable formulation.

The compounds of this invention may also be administered by inhalation, via the nasal or oral routes. Such administration can be carried out with a spray formulation comprising a compound of the invention and a suitable carrier, optionally suspended in, for example, a hydrocarbon propellant.

Preferred spray formulations comprise micronised compound particles in combination with a surfactant, solvent or a dispersing agent to prevent the sedimentation of suspended particles. Preferably, the compound particle size is from about 2 to 10 microns.

A further mode of administration of the compounds of the invention comprises transdermal delivery utilising a skin-patch formulation. A preferred formulation comprises a compound of the invention dispersed in a pressure sensitive adhesive which adheres to the skin, thereby permitting the compound to diffuse from the adhesive through the skin for delivery to the patient. For a constant rate of percutaneous absorption, pressure sensitive adhesives known in the art such as natural rubber or silicone can be used.

As mentioned above, the effective dose of compound depends on the particular compound employed, the condition of the patient and on the frequency and route of administration. A unit dose will generally contain from 20 to 1000 mg and preferably will contain from 30 to 500 mg, in particular 50, 100, 150, 200, 250, 300, 350, 400, 450, or 500 mg. The composition may be administered once or more times a day for example 2, 3 or 4 times daily, and the total daily dose for a 70 kg adult will normally be in the range 100 to 3000 mg. Alternatively the unit dose will contain from 2 to 20 mg of active ingredient and be administered in multiples, if desired, to give the preceding daily dose.

No unacceptable toxicological effects are expected with compounds of the invention when administered in accordance with the invention.

The present invention also provides a method for the treatment and/or prophylaxis of the Primary and Secondary Conditions in mammals, particularly humans, which comprises administering to the mammal in need of such treatment and/or prophylaxis an effective, non-toxic pharmaceutically acceptable amount of a compound of formula (I) or a pharmaceutically acceptable salt or solvate thereof.

The activity of the compounds of the present invention, as NK₃ ligands, is determined by their ability to inhibit the binding of the radiolabelled NK₃ ligands, [¹²⁵I]-[Me-Phe⁷]-NKB or [³H]-Senktide, to guinea-pig and human NK₃ receptors (Renzetti et al, **1991,** *Neuropeptide, 18,* 104-114; Buell et al, **1992,** *FEBS, 299(1),* 90-95; Chung et al, **1994**, *Biochem. Biophys. Res. Commun, 198(3),* 967-972).

The binding assays utilised allow the determination of the concentration of the individual compound required to reduce by 50% the [¹²⁵I]-[Me-phe⁷]-NKB and [³H]-Senktide specific binding to NK₃ receptor in equilibrium conditions (IC50).

Binding assays provide for each compound tested a mean IC₅₀ value of 2-5 separate experiments performed in duplicate or triplicate, The most potent compounds of the present invention show IC₅₀ values in the range 0.1-1000 nM. The NK₃-antagonist activity of the compounds of the present invention is determined by their ability to inhibit senktide-induced contraction of the guinea-pig ileum (Maggi et al, **1990,** *Br. J. Pharmacol., 101,* 996-1000) and rabbit isolated iris sphincter muscle (Hall et al., **1991,** *Eur. J. Pharmacol., 199,* 9-14) and human NK₃ receptors-mediated Ca⁺⁺ mobilisation (Mochizuki et al, **1994,** *J. Biol. Chem.,* 269, 9651-9658). Guinea-pig and rabbit *in-vitro* functional assays provide for each compound tested a mean K_{B} value of 3-8 separate experiments, where K_{B} is the concentration of the individual compound required to produce a 2-fold rightward shift in the concentration-response curve of senktide. Human receptor functional assay allows the determination of the concentration of the individual compound required to reduce by 50% (IC₅₀ values) the Ca⁺⁺ mobilisation induced by the agonist NKB. In this assay, the compounds of the present invention behave as antagonists.

The activity of the compounds of the present invention, as NK-2 ligands, is determined by their ability to inhibit the binding of the radiolabelled NK-2 ligands, [¹²⁵I]-NKA or [³H]-NKA, to human NK-2 receptors (Aharony et al, 1992, *Neuropeptide, 23,* 121-130).

The binding assays utilised allow the determination of the concentration of the individual compound required to reduce by 50% the [¹²⁵I]-NKA and [³H]-NKA specific binding to NK2 receptor in equilibrium conditions (IC₅₀).

Binding assays provide for each compound tested a mean IC₅₀ value of 2-5 separate experiments performed in duplicate or triplicate. The most potent compounds of the present invention show IC₅₀ values in the range 0.5-1000 nM, such as 1-1000 nM. The NK-2-antagonist activity of the compounds of the present invention is determined by their ability to inhibit human NK-2 receptor-mediated Ca⁺⁺ mobilisation (Mochizuki et al, **1994,** *J. Biol. Chem., 269, 9651-9658).* Human receptor functional assay allows the determination of the concentration of the individual compound required to reduce by 50% (IC₅₀ values) the Ca⁺⁺ mobilisation induced by the agonist NKA. In this assay, the compounds of the present invention behave as antagonists.

The therapeutic potential of the compounds of the present invention in treating the conditions can be assessed using rodent disease models.

As stated above, the compounds of formula (I) are also considered to be useful as diagnostic tools. Accordingly, the invention includes a compound of formula (I) for use as diagnostic tools for assessing the degree to which neurokinin-3 and neurokinin-2 receptor activity (normal, overactivity or underactivity) is implicated in a patient's symptoms. Such use comprises the use of a compound of formula (I) as an antagonist of said activity, for example including but not restricted to Tachykinin agonist-induced inositol phosphate turnover or electrophysiological activation, of a cell sample obtained from a patient. Comparison of such activity in the presence or absence of a compound of formula (I), will disclose the degree of NK-3 and NK-2 receptor involvement in the mediation of agonist effects in that tissue.

The following Descriptions illustrate the preparation of the intermediates, whereas the following Example illustrates the preparation of the compound of the invention.

### Descriptions and Example

### Description 1. 3-Methyl-2-pbenyl-quinoline-4-carboxylic acid methyl ester

30 g (114 mmol) of 3-methyl-2-phenyl-quinoline-4-carboxylic acid (CAS [43071-45-0]) were suspended in 250 ml of dry CH₂Cl₂; 20 ml (230 mmol) of oxalyl chloride dissolved in 120 ml of CH₂Cl₂ were added dropwise and the reaction mixture was stirred at room temperature for 30 min. Two drops of N,N-dimethylformamide (DMF) were added and the reaction was stirred for additional 30 min. The solvent was evaporated *in vacuo* to dryness, the residue was taken up with 100 ml of CH₂Cl₂ and 100 ml of MeOH, dissolved in 400 ml of CH₂Cl₂, were added dropwise. After stirring for 18 h, the solvent was evaporated *in vacuo* to dryness, the residue was taken up with CH₂Cl₂ and washed with 1% NaHCO₃; the organic layer was dried over Na₂SO₄, filtered and evaporated *in vacuo* to dryness to yield the title compound as a solid, which may be used without further purification.
C₁₈H₁₅NO₂
MW 277.31
MP = 73-75°C
IR (KBr) 3441, 3051,2954, 1731, 1582, 1556 cm⁻¹.

### Description 2. 3-Bromomethyl-2-phenyl-quinoline-4-carboxylic acid methyl ester

3-Methyl-2-phenyl-quinoline-4-carboxylic acid methyl ester (10 g, 36 mmol), prepared as in Description 1, was dissolved in CH₃CN (500 ml) and N-bromosuccinimide (13 g, 72 mmol) was added. After adding dibenzoylperoxide (1 g, 4.1 mmol), the reaction was refluxed for 24 h; then additional N-bromosuccinim.ide (4 g, 22.5 mmol) and dibenzoylperoxide (0.5 g, 2.0 mmol) were added and the reaction was refluxed for additional 4 h. The solvent was evaporated *in vacuo* to dryness to yield the tile compound, which may be used without further purification.
C₁₈H₁₄BrNO₂
MW = 356.23

### Description 3. 3-[1,4']Bipiperidinyl-1'-ylmethyl-2-phenyl-quinoline-4-carboxylic acid methyl ester

10.5 g (56.14 mmol) of 4-piperidinopiperidine and 7.8 g of K₂CO₃ were suspended in 350 ml CH₃CN. A solution of 20 g (56.14 mmol) 3-bromomethyl-2-phenyl-quinoline-4-carboxylic acid methyl ester (compound of Description 2) in 100 ml of CH₃CN/CH₂Cl₂ (10:1 mixture) was added dropwise at room temperature. The reaction was stirred overnight. After all the bromide had been consumed, the suspension was filtered and the obtained solution evaporated. The crude solid was taken up with i-Pr₂O and the insoluble portion was removed by filtration. The liquid phase was then evaporated to dryness and the resulting oil was purified by flash chromatography (eluent AcOEt:MeOH:NH₃ 95:5:0.5) to yield the title compound.
C₂₈H₃₃N₃O₂
MW = 443.59
MP =118-120°C

### Description 4. 3-[1,4']Bipiperidinyl-1'-ylmethyl-2-phenyl-quinoline-4-carboxylic acid

Aminoacid trihydrochloride (8.45 g, 15.67 mmol), obtained by hydrolysis in strongly acidic conditions of the corresponding 3-[1,4']bipiperidinyl-1'-ylmethyl-2-phenylquinoline-4-carboxylic acid methyl ester, was suspended in CH₃CN (150 ml) and 5.88 ml (0.918 g/ml, 47.04 mmol) of tetramethylguanidine were slowly added to the stirred mixture. By the end of the addition the aminoacid was completely dissolved and after a few minutes a white solid precipitated. The solid was filtered and crystallised from CH₃CN, yiedling a solid whose composition, established by ¹H-NMR, MS and IR analysis, resulted in the free aminoacid containing a ~20%_{WT} of tetramethylguanidine. In different batches tetramethylguanidine was variably detected, as hydrochloride, ranging from 8%_{WT} to 20%_{WT}. The presence of this salt however was found not to interfere with following the synthetic step.
C₂₇H₃₁N₃O₂
MW = 429.56

### Description 5. 8-Methyl-7-pbenyl-2,3-dihydro-[1,4]dioxino[2,3-g]quinoline-9-carboxylic acid

1,4-Benzodioxan-6-amine (10 g, 66 mmol) was dissolved in EtOH (200 ml) and benzaldehyde (6.7 ml, 66 mmol) was added. The solution was refluxed for 2 h and then 2-oxobutirric acid (6.7 g, 66 mmol) was added. The mixture was heated to reflux for additional 3 h and then cooled overnight. A solid precipitated, which was collected by suction, yielding the title compound, which may be used without further purification.
C₁₉H₁₅NO₄
MW= 321.33

### Description 6. 8-Methyl-7-phenyl-2,3-dihydro-[1,4]dioxino[2,3-g]quinoline-9-carboxylic acid ((S)-1-cyclohexyl-ethyl)-amide

8-Methyl-7-phenyl-2,3-dihydro-[1,4]dioxino[2,3-g]quinoline-9-carboxylic acid (2.25 g, 7 mmol), prepared as in Description 5, was suspended in CH₂Cl₂ (35 ml) and oxalyl chloride (1.3 ml, 15 mmol) was added dropwise at room temperature. After adding two drops of DMF, stirring was continued for 1h. Additional oxalyl chloride (0.7 ml, 7 mmol) was added to the solution and after 1 h the organic solvent was evaporated to dryness. The solid was dissolved in CH₂Cl₂ and added dropwise to a suspension of (S)-1-cyclohexylethylamine (2.15 ml, 14 mmol) and K₂CO₃ (2.9 g, 21 mmol) in CH₂Cl₂ (30 ml). The reaction was refluxed for 24 h, cool to room temperature and the solvent evaporated to dryness. The crude solid was dissolved in AcOEt and washed with NaOH, HCl, and brine. The organic phase was dried on Na₂SO₄, filtered and evaporated to dryness obtaining a crude residue that, when triturated with Et₂O, gave the title compound as a solid.
C₂₇H₃₀N₂O₃
MW = 430.55
MP= 217-219°C

### Description 7. 7-Methyl-6-phenyl-[1,3]dioxolo[4,5-g]quinoline-8-carboxylic acid

3,4-Methylenedioxyaniline (20.16 g, 147 mmol) was dissolved in EtOH (300 ml) and both benzaldehyde (14.3 ml, 147 mmol) and 2-oxobutirric acid (15 g, 147 mmol) were added. The solution was stirred at room temperature for three days. A solid was formed which was collected by filtration and dissolved in NaOH 1 M, The solution was washed with Et₂O and acidification of the aqueous phase a solid precipitated. The solid was filtered by suction and dried in vacuum oven to yield the title compound as a solid.
C₁₈H₁₃NO₄
MW = 307.30
MP=>300°C

### Description 8. 7-Methyl-6-phenyl-[1,3]dioxolo[4,5-g]quinoline-8-carboxylic acid ((S)-1-cyclohexyl-ethyl)-amide

7-Methyl-6-phenyl-[1,3]dioxolo[4,5-g]quinohne-8-carboxylic acid (10 g, 32.5 mmol), prepared as in Description 7, was suspended in CH₂Cl₂ (200 ml) and cooled to 0-5°C. Oxalyl chloride (5.8 ml, 65 mmol) was added dropwise under stirring in 15 min. After adding few drops of DMF, the mixture was allowed to warm to room temperature and left for 3 h. The organic solvent was evaporated to dryness. The crude residue was dissolved with CH₂Cl₂ and added dropwise to a stirred suspension of (S)-1-cyclohexylethylamine (5.8 ml, 39.05 mmol) and K₂CO₃ (9 g) in CH₂Cl₂ (150 ml). The solid was filtered and the organic solvent was evaporated to dryness. The crude residue was purified by flash chromatography (eluent hexane:AcOEt 6:4) to afford the title compound as a solid.
C₂₆H₂₈N₂O₃
MW = 416.52

### Description 9. (3-Amino-phenyl)-methanol

The suspension of LiA1H₄ (10 g, 263 mmol) in dry THF (500 ml) was cooled to 0°C and a solution of m-aminobenzoic acid (17.83 g, 130 mmol) in dry THF (500 ml) was slowly added dropwise maintaining the internal temperature as constant as possible and using mechanical stirring. After the end of the addition, the suspension was refluxed for three days. H₂O (10 ml) was carefully added followed by 15% NaOH (30 ml) and H₂O (10 ml). The suspension was filtered and the organic phase was dried over Na₂SO₄, filtered and evaporated to dryness to afford the title compound, which may be used without further purification.
C₇H₉NO
MW = 123.15

### Description 10. 7-Hydroxymethyl-3-methyl-2-phenyl-quinoline-4-carboxylic acid

To a solution of crude (3-amino-phenyl)-methanol (18 g), prepared as in Description 9, in EtOH (400 ml) benzaldehyde (14.2 ml, 140 mmol) was added. The solution was refluxed for 2 h and then 2-oxobutirric acid (14.3 g, 140 mmol) was added. The mixture was refluxed for 3 h. The reaction was allowed to cool overnight to room temperature and the resulting precipitate was collected by filtration to afford the title compound.
C₁₈H₁₅NO₃
MW = 293.32

### Description 11. 7-Hydroxymethyl-3-methyl-2-phenyl-quinoline-4-carboxylic acid ((S)-1-cyclohexyl-ethyl)-amide

7-Hydroxymethyl-3-methyl-2-phenyl-quinoline-4-carboxylic acid (6,75 g, 23 mmol), prepared as in Description 10, was suspended in THF:CH₂Cl₂ mixture (300 ml) and triethylamine (12.8 ml, 92 mmol) and HBTU (8.75 g, 23 mmol) were added. After heating the solution to 40°C until it becomes clear (about 30 min), (S)-1-cyclohexylethylamine (6.5 ml, 46 mmol) was added and the solution kept at 40°C for 3 h. The reaction was allowed to cool overnight to room temperature. The organic solvent was evaporated to dryness and the resulting crude residue was dissolved in AcOEt, washed with water, HCl (1 M), NaOH (1 M) and then with water. The organic phase was dried over Na₂SO₄, filtered and evaporated to dryness obtaining an oil that, when treated with Et₂O, yielded the title compound as a solid.
C₂₅H₂₈N₂O₂
MW = 388.51
MP= 160-161°C

### Description 12. 4-((S)-1-Cyclohexyl-ethylcarbamoyl)-3-methyl-2-phenyl-quinoline-7-carboxylic acid

A solution of 7-hydroxymethyl-3-methyl-2-phenyl-quinoline-4-carboxylic acid ((S)-1-cyclohexyl-ethyl)-amide (5 g, 12.5 mmol), prepared as in Description 11, in THF (1.25 L) was added dropwise aqueous 0.2 M KOH (2.5 L), containing K₂S₂O₈ (20.27 g, 75 mmol) and RuCl₃ (881 mg, 4.25 mmol). The resulting mixture was stirred at room temperature for 1.5 h. THF was then removed and the remaining aqueous solution washed with Et₂O. The aqueous phase was acidified with 1M HCl and extracted with AcOEt. The organic phase was then dried over Na₂SO₄, filtered and evaporated to dryness to afford the crude product. The crude product was triturated with Et₂O to afford the title compound.
C₂₆H₂₈N₂O₃
MW = 416.52
MP= 70°C

### Description 13. 5-[1,4']Bipiperidinyl-1'-yl-1-phenyl-pentan-1-one

Mesyl chloride (2.5 ml, 32 mmol) in DCM (10 ml) was added dropwise at 0°C to a solution of 5-hydroxy-1-phenyl-pentan-1-one (5 g, 28 mmol, CAS[1011-62-7]), prepared as in Description 12, and Et₃N (5 ml, 36 mmol) in DCM (65 ml). The reaction was left 30 min at 0°C and then 1h at room temperature. The organic layer was washed with H₂O, dried over Na₂SO₄ and then evaporated to dryness. The crude mesylate was dissolved in DMF (20 ml) and 4-piperidinopiperidine (4.7 g, 28 mmol) and Et₃N (3.9 ml, 28 mmol) were added. The reaction was stirred at 60°C for 18 h. The organic solvent was removed under reduced pressure and the crude compound was purified by column chromatography (AcOEt/ MeOH 7:3 and then 1:1) to afford the title compound as an oil.

### Description 14. 3-(3-[1,4']Bipiperidinyl-1'-yl-propyl)-2-phenyl-quinoline-4-carboxylie acid

A suspension of isatine (2.24 g, 15.2 mmol) and KOH (3.42 g, 61 mmol) in EtOH (200 ml) was stirred for 1.5 h at room temperature. 5-[1,4']Bipiperidinyl-1'-yl-1-phenyl pentan-1-one (5 g, 15.2 mmol), prepared as in Description 13, was added and the reaction was refluxed for 6 days. The organic solvent was removed under vacuum and the residue was dissolved in H₂O, acidified with HCl to pH 1 and then extracted with DCM, The organic phase was dried over Na₂SO₄ and evaporated to dryness to afford the title compound.

### Description 15: 6,7-Dimethoxy-3-methyl-2-phenyl-quinoline-4-carboxylic acid ((S)-1-cyclohexyl-ethyl)-amide

This compound was prepared starting from 3,4-dimethoxyaniline and following the procedure described in Description 7-8.

### Description 16: 3-Bromomethyl-6,7-dimethoxy-2-phenyl-quinoline-4-carboxylic acid ((S)-1-cyclohexyl-ethyl)-amide

6,7-Dimethoxy-3-methyl-2-phenyl-quinoline-4-carboxylic acid ((S)-1-cyclohexyl-ethyl)-amide (0.7 g, 1.62 mmol), prepared as in Description 15, was suspended in CH₃CN (40 ml) and warmed to 50°C. N-Bromosuccinimide (1.44 g, 8.1 mmol) and dibenzoylperoxide (30 mg) were added. The reaction was refluxed for 4 h. The organic solvent was removed under reduced pressure to afford the title compound which may be used without further purification.

### Description 17. 1-(4-Methoxy-cyclohexyl)-ethylamine hydrochloride

(S)-1-(4-Methoxyphenyl)ethylamine (1 g, 6 mmol), dissolved in EtOH (20 ml) containing acetic acid (1 ml), was hydrogenated over Rh/Al₂O₃ at 55 psi for 16 h at room temperature. The catalyst was filtered off and the organic solvent was evaporated to dryness. The residue was dissolved in EtOAc, acidified with HCl and evaporated to dryness. This operation was repeated several times to afford the tile compound which may be without further purification.

### Description 18: 1-(2-Fluoro-phenyl)-propylamine

2-Fluoropropiophenone (1.5 g, 9-86 mmol) and O-methylhydroxylamina hydrochloride (1.5 g, 20 mmol) were dissolved in EtOH/H₂O 3:1 (15 ml). 32% NaOH (2.1 ml) was added dropwise. The reaction was stirred overnight at room temperature and then refluxed for 4 h. The organic solvent was removed under reduced pressure and the residue was dissolved in Et₂O, washed with water, dried over Na₂SO₄, filtered and evaporated to dryness obtaining 1.35 g of the crude O-methyl-oxime intermediate. This compound was dissolved in anhydrous THF (15 ml) and 1M BH₃ in THF (7 ml) was added at 0°C. The reaction was refluxed for 3h. After cooling to 0°C, H2O (10 ml) and 20% KOH (10 ml) were added dropwise. The reaction was refluxed for about 2 h. The organic solvent was removed under reduced pressure and the residue was dissolved in Et₂O and extracted with 2M HCl. The aqueous phase was basified with 1M NaOH and extracted with Et₂O. The organic phase was dried over Na₂SO₄, filtered and evaporated to dryness to afford the title compound, which may be used without further purification.

### Description 19: 7-Methyl-6-phenyl-[1,3]dioxolo[4,5-g]quinoline-8-carboxylic acid methyl ester

7-Methyl-6-phenyl-[1,3]dioxolo[4,5-g]quinoline-8-carboxylic acid (10 g, 32.5 mmol), prepared as in Description 7, was suspended in CH₂Cl₂ (200 ml) and cooled to 0-5°C. Oxalyl chloride (5.8 ml, 65 mmol) was added dropwise under stirring in 15 min. After adding few drops of DMF, the mixture was allowed to warm to room temperature and left for 3 h. The organic solvent was evaporated to dryness. The crude residue was dissolved in MeOH and refluxed for 4 h. The organic solvent is evaporated to dryness to afford the title compound.

### Description 20: 7-Broamonnethyl-6-phenyl-[1,3]dioxolo[4,5-g]quinoline-8-carboxylic acid methyl ester

To a solution of 7-methyl-6-phenyl-[1,3]dioxolo[4,5-g]quinoline-8-carboxylic acid methyl ester (8.27 g, 25.74 mmol), prepared as in Description 19, in CH₃CN (400 ml), N-bromosuccimide (18.32 g, 102.25 mmol) and benzoylperoxide (0.3 g) were added. The reaction was carefully heated under reflux for 5 h. N-bromosuccimide (4.6 g, 25.5 mmol) and benzoylperoxide (0.3 g) were added and the reaction was refluxed overnight. The organic solvent was removed under reduced pressure and the crude reaction product was used in the next step without further purification.

### Description 21: 7-[1,4']Bipipendinyl-1'-yhnethyl-6-phenyl-[1,3]dioxolo[4,5-g]quinoline-8-carboxylic acid methyl ester

The crude 7-bromomethyl-6-phenyl-[1,3]dioxolo[4,5-g]quinoline-8-carboxylic acid methyl ester, prepared as in Description 20, was dissolved in CH₃CN (100 ml) and added dropwise to a suspension of K₂CO₃ (14.23 g) and 4-piperidanopiperidine (9.6 g, 588 mmol) in CH₃CN (300 ml). The reaction was refluxed for 4 h. The organic solvent was removed under reduced pressure and the residue was re-dissolved in EtOAc, washed with 0.5N HCl, dried over Na₂SO₄, filtered and evaporated to dryness. The solid is triturated with Et₂O to give, after filtration, the title compound together with other brominated quinoline derivatives.
Compound prepared as above dissolved in AcOEt/THF 1:1 (125 ml), was hydrogenated over 10% Pd/C (480 mg) at 6 psi for 70 h at room temperature. The catalyst was filtered off and the organic solvent was evaporated to dryness to afford the title compound.

### Description 22: 7-[1,4']Bipiperidioyl-1'-ylmethyl-6-phenyl-[1,3]dioxolo[4,5-g]quinoline-8-carboxcylic acid hydrochloride

7-[1,4']Bipiperidinyl-1'-ylmethyl-6-phenyl-[1,3]dioxolo[4,5-g]quinoline-8-carboxylic acid methyl ester (0.5 g, 1 mmol) was dissolved in 6M HCl and refluxed for 1.5 h. The reaction was evaporated to dryness, dissolved in CH₃CN and evaporated to dryness. This operation was repeated several times to afford the title compound as a solid.

### Example 1: 8-[1,4']Bipiperidinyl-1'-ylmethyl-7-phenyl-2,3-dihydro-[1,4]dioidmo[2,3-glquinoline-9-carboxylic acid ((S)-1-cyclohexyl-ethyl)-amide

8-Methyl-7-phenyl-2,3-dihydro-[1,4]dioxino[2,3-g]quinoline-9-carboxylic acid ((S)-1-cyclohexyl-ethyl)-amide (1.07 g, 2.5 mmol), prepared as in Description 6, was suspended in CCl₄ (40 ml) and NBS (0.89 g, 5 mmol) was added. The mixture was heated to reflux and 10 mg of benzoylperoxide were added. After 1 hour the mixture had cleared and all the starting material had been consumed. A 10% of dibrominated compound was also detected. The solvent was evaporated and the residue was taken up with CH₃CN (50 ml). The solution was added dropwise to a suspension of 4-piperidinopiperidine (0.84 g, 5 mmol) and K₂CO₃ (0.7 g, 5 mmol) in CH₃CN (50 ml). The reaction was refluxed for 1 h. The mixture was allowed to cool to room temperature, filtered and the organic solvent evaporated to dryness. The oil was purified by column cromatography (eluent CH₂Cl₂/MeOH/NH₃ 95/5/0.5) to afford the title compound as a solid.

**Table 1 (Example)**

| | | | |
|---|---|---|---|
| 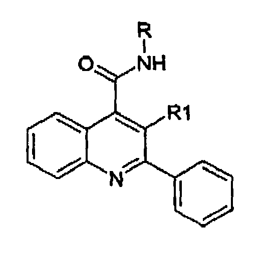 | | | |

| **Example** | **R** | **R1** | **Molecular formula** |
|---|---|---|---|
| 1 | 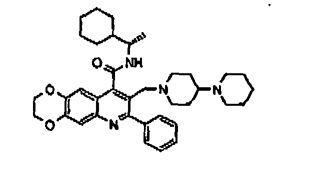 | | C₃₇H₄₈N₄O₃ |

**Table 2**

| **¹H NMR spectroscopy data of Example 1** | |
|---|---|
| **Ex** | **¹H NMR (Solvent) ppm** |
| 1 | ¹H NMR (DMSO, 343 K) δ: 8.22 (d br, 1H); 7.51 (m, 2H); 7.47-7.39 (m, 3H); 7.37 (s, 1H); 7.20 (s, 1H); 4.39 (s, 4H); 4.01 (m, 1H); 3.50 (s, 2H); 2.52 (m, 2H); 2.36 (m, 4H); 1.99 (m, 1H); 1.88-1.60 (m, 7H); 1.53-1.05 (m, 16H); 1.18 (d, 3H). EI; TSQ 700; source 180°C; 70 V; 200 uA: 596 (M+.); 430; 277; 167; 124 |

**Table 3**

| **Chemical names of parent compounds of Example 1 (names generated bv Beilstein's Autonom)** | |
|---|---|
| **Ex** | **Chemical name** |
| 1 | 8-[1,4']Bipiperidinyl-1'-ylmethyl-7-phenyl-2,3-dihydro-[1,4]dioxino[2,3-g]quinoline-9-carboxylic acid ((S)-1-cyclohexyl-ethyl)-amide |

## Claims

1. 8-[1,4']Bipiperidinyl-1'-ylmethyl-7-phenyl-2,3-dihydro-[1,4]dioxino[2,3-g]quinoline-9-carboxylic acid ((S)-1-cyclohexyl-ethyl)-amide or a pharmaceutically acceptable salt or solvate thereof.

2. A pharmaceutical composition comprising a compound according to claim 1 and a pharmaceutically acceptable carrier.

3. A compound according to claim 1 for use as an active therapeutic substance.

4. A compound according to claim 1 or a pharmaceutically acceptable salt or solvate thereof, for the treatment or prophylaxis of respiratory diseases, such as chronic obstructive pulmonary disease (COPD), asthma, airway hyper-reactivity, cough; inflammatory diseases such as inflammatory bowel disease, psoriasis, fibrositis, osteoarthritis, rheumatoid arthritis and inflammatory pain; neurogenic inflammation or peripheral neuropathy, allergies such as eczema and rhinitis; ophthalmic diseases such as ocular inflammation, conjunctivitis, vernal conjuctivitis and the like; cutaneous diseases, skin disorders and itch, such as cutaneous wheal and flare, contact dermatitis, atopic dermatitis, urticaria and other eczematoid dermatitis; adverse immunological reactions such as rejection of transplanted tissues and disorders related to immune enhancement or suppression such as systhemic lupus erythematosis; gastrointestinal (GI) disorders and diseases of the GI tract such as disorders associated with the neuronal control of viscera such as ulcerative colitis, Crohn's disease, irritable bowel syndrome (IBS), gastro-exophageous reflex disease (GERD); urinary incontinence and disorders of the bladder function; renal disorders; increased blood pressure, proteinuria, coagulopathy and peripheral and cerebral oedema following pre-eclampsia in pregnancies; disorders of the central nervous system such as anxiety, depression, psychosis and schizophrenia; neurodegenerative disorders such as AIDS related dementia, senile dementia of the Alzheimer type, Alzheimer's disease, Down's syndrome, Huntingdon's disease, Parkinson's disease, movement disorders and convulsive disorders (for example epilepsy); demyelinating diseases such as multiple sclerosis and amyotrophic lateral sclerosis and other neuropathological disorders such as diabetic neuropathy, AIDS related neuropathy, chemotherapy-induced neuropathy and neuralgia; addiction disorders such as alcoholism; stress related somatic disorders; reflex sympathetic dystrophy such as shoulder/hand syndrome; dysthymic disorders; eating disorders (such as food intake disease); fibrosing and collagen diseases such as scleroderma and eosinophilic fascioliasis; disorders of the blood flow caused by vasodilatation and vasospastic diseases such as angina, migraine and Reynaud's disease and pain or nociception, for example, that is attributable to or associated with any of the foregoing conditions especially the transmission of pain in migraine.

5. Use of a compound according to claim 1 or a pharmaceutically acceptable salt or solvate thereof, in the manufacture of a medicament for the treatment of respiratory diseases, such as chronic obstructive pulmonary disease (COPD), asthma, airway hyper-reactivity, cough; inflammatory diseases such as inflammatory bowel disease, psoriasis, fibrositis, osteoarthritis, rheumatoid arthritis and inflammatory pain; neurogenic inflammation or peripheral neuropathy, allergies such as eczema and rhinitis; ophthalmic diseases such as ocular inflammation, conjunctivitis, vernal conjuctivitis and the like; cutaneous diseases, skin disorders and itch, such as cutaneous wheal and flare, contact dermatitis, atopic dermatitis, urticaria and other eczematoid dermatitis; adverse immunological reactions such as rejection of transplanted tissues and disorders related to immune enhancement or suppression such as systhemic lupus erythematosis; gastrointestinal (GI) disorders and diseases of the GI tract such as disorders associated with the neuronal control of viscera such as ulcerative colitis, Crohn's disease, irritable bowel syndrome (IBS), gastro-exophageous reflex disease (GERD); urinary incontinence and disorders of the bladder function; renal disorders; increased blood pressure, proteinuria, coagulopathy and peripheral and cerebral oedema following pre-eclampsia in pregnancies; disorders of the central nervous system such as anxiety, depression, psychosis and schizophrenia; neurodegenerative disorders such as AIDS related dementia, senile dementia of the Alzheimer type, Alzheimer's disease, Down's syndrome, Huntingdon's disease, Parkinson's disease, movement disorders and convulsive disorders (for example epilepsy); demyelinating diseases such as multiple sclerosis and amyotrophic lateral sclerosis and other neuropathological disorders such as diabetic neuropathy, AIDS related neuropathy, chemotherapy-induced neuropathy and neuralgia; addiction disorders such as alcoholism; stress related somatic disorders; reflex sympathetic dystrophy such as shoulder/hand syndrome; dysthymic disorders; eating disorders (such as food intake disease); fibrosing and collagen diseases such as scleroderma and eosinophilic fascioliasis; disorders of the blood flow caused by vasodilatation and vasospastic diseases such as angina, migraine and Reynaud's disease and pain or nociception, for example, that is attributable to or associated with any of the foregoing conditions especially the transmission of pain in migraine.

## Patentansprüche

1. 8-[1,4']Bipiperidinyl-1'-ylmethyl-7-phenyl-2,3-dihydro[1,4]dioxin[2,3-g]chinolin-9-carbonsäure((S)-1-cyclohexylethyl)amid oder ein pharmazeutisch verträgliches Salz oder Solvat davon.

2. Arzneimittel, umfassend eine Verbindung gemäß Anspruch 1 und einen pharmazeutisch verträglichen Träger.

3. Verbindung gemäß Anspruch 1 zur Verwendung als therapeutischer Wirkstoff.

4. Verbindung gemäß Anspruch 1 oder ein pharmazeutisch verträgliches Salz oder Solvat davon zur Behandlung oder Prophylaxe von respiratorischen Krankheiten, wie chronisch obstruktive Lungenkrankheit (COPD), Asthma, Atemwegshyperreaktivität, Husten; Entzündungskrankheiten, wie entzündliche Krankheit im oberen Verdauungstrakt, Psoriasis, Fibrositis, Osteoarthritis, rheumatoide Arthritis und Entzündungsschmerz; neurogener Entzündung oder peripherer Neuropathie, Allergien, wie Ekzem und Rhinitis; Augenkrankheinen, wie Augenentzündung, Conjunktivitis, Conjunktivitis vernalis und dergleichen; Hautkrankheiten, Hautstörungen und Krätze, wie Hautquaddel und Hautrötung, Kontaktdermatitis, atopische Dermatitis, Urticaria und andere ekzematoide Dermatitis; immunologischen Abwchrreaktionen, wie Abstoßung transplantierter Gewebe und Störungen, bedingt durch Immunsteigerung oder Immunsuppression, wie systemische Lupuserythematodes; gastrointestinalen (GI) Störungen und Krankheiten des GI-Trakts, wie Störungen, die in Zusammenhang mit neuronaler Kontrolle der Eingeweide stehen, wie ulzerative Colitis, Morbus Crohn, Reizdarm (IBS), gastroösophageale Refluxkrankheit (GERD); Harninkontinenz und Störungen der Blasenfunktion; Nierenerkrankungen; erhöhtem Blutdruck, Proteinurie, Gerinnungsdefekt und peripherem und cerebralem Ödem nach Präeklampsie in Schwangerschaften; Störungen des zentralen Nervensystems, wie Angst, Depression, Psychose und Schizophrenie; neurodegenerativen Störungen, wie AIDS bedingte Demenz, senile Demenz vom Alzheimer-Typ, Alzheimer-Krankheit, Down-Syndrom, Huntington-Krankheit, Parkinson-Krankheit, Bewegungsstörungen und konvulsive Störungen (zum Beispiel Epilepsie); demyelinisierenden Erkrankungen, wie Multiple Sklerose und amyotrophe Lateralsklerose und andere neuropathologische Krankheiten, wie diabetische Neuropathie, AIDS bedingte Neuropathie, Chemotherapie-induzierte Neuropathie und Neuralgie; Abhärgigkeitsstörungen, wie Alkoholismus; stressbedingten somatischen Störungen; Algodystrophie-Syndrom, wie Schulter/Hand-Syndrom; dysthymischen Störungen; Essstörungen (wie Nahrungsaufnahme-Erkrankung); Fibrosen und Kollagenosen, wie Sklerodermie und eosinophile Fascioliasis; Störungen des Blutflusses verursacht durch Vasodilatation und vasospastische Krankheiten, wie Angina, Migräne und Raynaud-Krankheit und Schmerz oder Nozizeption, zum Beispiel jene, die auf einen der vorstehenden Zustände, insbesondere die Transmission von Schmerz bei Migräne, zurückzuführen ist oder mit jener in Zusammenhang steht.

5. Verwendung einer Verbindung gemäß Anspruch 1 oder eines pharmazeutisch verträglichen Salzes oder Solvats davon bei der Herstellung eines Medikaments zur Behandlung von respiratorischen Krankheiten, wie chronisch obstruktive Lungenkrankheit (COPD), Asthma. Atemwegshyperreaktivität, Husten; Entzündungskrankheiten, wie entzündliche Krankheit im oberen Verdauungstrakt, Psoriasis, Fibrositis, Osteoarthritis, rheumatoide Arthritis und Entzündungsschmerz; neurogener Entzündung oder peripherer Neuropathie, Allergien, wie Ekzem und Rhinitis; Augenkrankheiten, wie Augenentzündung, Conjunktivitis, Conjunktivitis vernalis und dergleichen; Hautkrankheiten, Hautstörungen und Krätze, wie Hautquaddel und Hautrötung, Kontaktdermatitis, atopische Dermatitis, Urticaria und andere ekzematoide Dermatitis; immunologischen Abwehrreaktionen, wie Abstoßung transplantierter Gewebe und Störungen, bedingt durch Immunsteigerung oder Immunsuppression, wie systemische Lupuserythematodes; gastrointestinalen (GI) Störungen und Krankheiten des GI-Trakts, wie Störungen, die in Zusammenhang mit neuronaler Kontrolle der Eingeweide stehen, wie ulzerative Colitis, Morbus Crohn, Reizdarm (IBS), gastroösophageale Refluxkrankheit (GERD); Harninkontinenz und Störungen der Blasenfunktion; Nierenerkrankungen; erhöhtem Blutdruck, Proteinurie, Gerinnungsdefekt und peripherem und cerebralem Ödem nach Präeklampsie in Schwangerschaften; Störungen des zentralen Nervensystems, wie Angst, Depression, Psychose und Schizophrenie; neurodegenerativen Störungen, wie AIDS bedingte Demenz, senile Demenz vom Alzheimer-Typ, Alzheimer-Krankheit, Down-Syndrom. Huntington-Krankheit, Parkinson-Krankheit, Bewegungsstörungen und konvulsive Störungen (zum Beispiel Epilepsie); demyelinisierenden Erkrankungen, wie Multiple Sklerose und amyotrophe Lateralsklerose und andere neuropathologische Krankheiten, wie diabetische Neuropathie, AIDS bedingte Neuropathie, Chemotherapie-induzierte Neuropathie und Neuralgie; Abhängigkeitsstörungen, wie Alkoholismus; stressbedingten somatischen Störungen; Algodystrophie-Syndrom, wie Schulter/Hand-Syndrom; dysthymischen Störungen; Essstörungen (wie Nahrungsaufnahme-Erkrankung); Fibrosen und Kollagenosen, wie Sklerodermie und eosinophile Fascioliasis; Störungen des Blutflusses verursacht durch Vasodilatation und vasospastische Krankheiten, wie Angina, Migräne und Raynaud-Krankheit und Schmerz oder Nozizeption, zum Beispiel jene, die auf eine der vorstehenden Zustände, insbesondere die Transmission von Schmerz bei Migräne, zurückzuführen ist oder mit jener in Zusammenhang steht.

## Revendications

1. ((S)-1-cyclohexyl-éthyl)-amide d'acide 8-[1,4']-bipipéridinyl-1'-ylméthyl-7-phényl-2,3-dihydro-[1,4]-dioxino[2,3-g]quinoléine-9-carboxylique, ou un de ses sels ou produits de solvatation pharmaceutiquement acceptables.

2. Composition pharmaceutique comprenant un composé suivant la revendication 1 et un support pharmaceutiquement acceptable.

3. Composé suivant la revendication 1, destiné à être utilisé comme substance thérapeutique active.

4. Composé suivant la revendication 1 ou un de ses sels ou produits de solvatation pharmaceutiquement acceptables, pour le traitement ou la prophylaxie de maladies respiratoires, comme la maladie pulmonaire obstructive chronique (COPD), l'asthme, l'hyperactivité des voies aériennes, la toux ; de maladies inflammatoires comme la maladie intestinale inflammatoire, le psoriasis, la fibrosite, l'arthrose, la polyarthrite rhumatoïde et la douleur inflammatoire ; d'inflammations neurogènes ou de la neuropathie périphérique, d'allergies comme l'eczéma et la rhinite ; de maladies ophtalmiques comme l'inflammation oculaire, la conjonctivite, la conjonctivite printanière et des maladies similaires ; de maladies cutanées, de troubles cutanés et du prurit, comme la boule d'oedème et d'érythème cutanés, la dermatite de contact, la dermatite atopique, l'urticaire et d'autres dermatites eczématoïdes ; de réactions immunologiques néfastes comme le rejet de tissus transplantés et de troubles en rapport avec une stimulation ou suppression immunitaire comme le lupus érythémateux disséminé ; de troubles gastro-intestinaux (GI) et de maladies du tractus GI comme les troubles associés au contrôle neuronal des viscères comme la colite ulcérative, la maladie de Crohn, le syndrome du côlon irritable (IBS), la maladie de reflux gastro-oesophagien (GERD) ; de l'incontinence urinaire et de troubles de la fonction vésicale ; de troubles rénaux ; de l'élévation de la pression sanguine, de la protéinurie, de la coagulopathie et de l'oedème périphérique et cérébral après la prééclampsie dans la grossesse ; de troubles du système nerveux central comme l'anxiété, la dépression, la psychose et la schizophrénie ; de troubles neurodégénératifs comme la démence liée au SIDA, la démence sénile de type Alzheimer, la maladie d'Alzheimer, le syndrome de Down, la maladie d'Huntington, la maladie de Parkinson, de troubles du mouvement et de troubles convulsifs (par exemple l'épilepsie) ; de maladies démyélinisantes comme la sclérose en plaques et la sclérose latérale amyotrophique, et d'autres troubles neuropathiques comme la neuropathie diabétique, la neuropathie due au SIDA, la neuropathie et les névralgies induites par la chimiothérapie ; de troubles d'accoutumance comme l'alcoolisme ; de troubles somatiques dus au stress ; de la dystrophie sympathique réflexe comme le syndrome épaule/main ; de troubles dysthymiques ; de troubles de l'alimentation (comme une maladie d'absorption de la nourriture) ; de maladies comportant une fibrose et de maladies du collagène comme la sclérodermie et la fasciolase éosinophile; de troubles du flux sanguin provoqués par une vasodilatation et de maladies angiospastiques comme l'angine de poitrine, la migraine et la maladie de Reynaud, et de la douleur ou de la nociception, par exemple, qui est attribuable ou associée à n'importe lequel des états précités, notamment la transmission de la douleur dans le cas de la migraine.

5. Utilisation d'un composé suivant la revendication 1, ou d'un de ses sels ou produits de solvatation pharmaceutiquement acceptables, dans la production d'un médicament destiné au traitement de maladies respiratoires comme la maladie pulmonaire obstructive chronique (COPD), l'asthme, l'hyperactivité des voies aériennes, la toux ; de maladies inflammatoires comme la maladie intestinale inflammatoire, le psoriasis, la fibrosite, l'arthrose, la polyarthrite rhumatoïde et la douleur inflammatoire ; d'inflammations neurogènes ou de la neuropathie périphérique, d'allergies comme l'eczéma et la rhinite ; de maladies ophtalmiques comme l'inflammation oculaire, la conjonctivite, la conjonctivite printanière et des maladies similaires ; de maladies cutanées, de troubles cutanés et du prurit, comme la boule d'oedème et d'érythème cutanés, la dermatite de contact, la dermatite atopique, l'urticaire et d'autres dermatites eczématoïdes ; de réactions immunologiques néfastes comme le rejet de tissus transplantés et de troubles en rapport avec une stimulation ou suppression immunitaire comme le lupus érythémateux disséminé ; de troubles gastro-intestinaux (GI) et de maladies du tractus GI comme les troubles associés au contrôle neuronal des viscères comme la colite ulcérative, la maladie de Crohn, le syndrome du côlon irritable (IBS), la maladie de reflux gastro-oesophagien (GERD) ; de l'incontinence urinaire et de troubles de la fonction vésicale ; de troubles rénaux ; de l'élévation de la pression sanguine, de la protéinurie, de la coagulopathie et de l'oedème périphérique et cérébral après la prééclampsie dans la grossesse ; de troubles du système nerveux central comme l'anxiété, la dépression, la psychose et la schizophrénie ; de troubles neurodégénératifs comme la démence liée au SIDA, la démence sénile de type Alzheimer, la maladie d'Alzheimer, le syndrome de Down, la maladie d'Huntington, la maladie de Parkinson, de troubles du mouvement et de troubles convulsifs (par exemple l'épilepsie) ; de maladies démyélinisantes comme la sclérose en plaques et la sclérose latérale amyotrophique, et d'autres troubles neuropathiques comme la neuropathie diabétique, la neuropathie due au SIDA, la neuropathie et les névralgies induites par la chimiothérapie ; de troubles d'accoutumance comme l'alcoolisme ; de troubles somatiques dus au stress ; de la dystrophie sympathique réflexe comme le syndrome épaule/main ; de troubles dysthymiques ; de troubles de l'alimentation (comme une maladie d'absorption de la nourriture) ; de maladies comportant une fibrose et de maladies du collagène comme la sclérodermie et la fasciolase éosinophile ; de troubles du flux sanguin provoqués par une vasodilatation et de maladies angiospastiques comme l'angine de poitrine, la migraine et la maladie de Reynaud, et de la douleur ou de la nociception, par exemple, qui est attribuable ou associée à n'importe lequel des états précités, notamment la transmission de la douleur dans le cas de la migraine.
